(19) **Europäisches Patentamt / European Patent Office / Office européen des brevets**

(11) **EP 3 467 505 A1**

(12) **EUROPEAN PATENT APPLICATION**

(43) Date of publication:
10.04.2019 Bulletin 2019/15

(51) Int Cl.:
*G01N 33/68* (2006.01)

(21) Application number: 17194837.5

(22) Date of filing: 04.10.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Fraunhofer Gesellschaft zur Förderung der angewandten Forschung e.V.**
**80686 München (DE)**
• **Johann Wolfgang Goethe-Universität Frankfurt**
**60323 Frankfurt am Main (DE)**

(72) Inventors:
• **LÖTSCH, Jörn**
**60590 Frankfurt am Main (DE)**
• **GEISSLINGER, Gerd**
**65812 Bad Soden (DE)**
• **TEGEDER, Irmgard**
**60598 Frankfurt (DE)**
• **SCHIFFMANN, Irmgard**
**63263 Neu-Isenburg (DE)**

(74) Representative: **Herzog, Fiesser & Partner Patentanwälte PartG mbB**
**Dudenstrasse 46**
**68167 Mannheim (DE)**

(54) **LIPID BASED BIOMARKER FOR MULTIPLE SCLEROSIS**

(57) The present invention relates to a method for diagnosing multiple sclerosis (MS) in a subject suspected to suffer therefrom comprising the steps of: a) determining the amounts of at least three markers from a group of markers comprising LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) in a sample of said subject; b) comparing the determined amounts to a reference or to references; and c) diagnosing MS based on said comparison in said subject.

**EP 3 467 505 A1**

Printed by Jouve, 75001 PARIS (FR)

**Description**

The present invention relates to a method for diagnosing multiple sclerosis (MS) in a subject suspected to suffer therefrom comprising the steps of: a) determining the amounts of at least three markers from a group of markers comprising LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) in a sample of said subject; b) comparing the determined amounts to a reference or to references; and c) diagnosing MS based on said comparison in said subject.

Multiple sclerosis (MS) is a chronic inflammatory, demyelinating and neurodegenerative autoimmune disease that affects the central nervous system. In the most frequent relapsing-remitting form (RRMS) symptomatic periods alternate with longer periods of remission at disease onset but may eventually turn into a secondary progressive disease (Compston and. Coles, Multiple sclerosis, Lancet 372(9648) (2008) 1502-17). Hence, the disease course is mostly characterized by a worsening of non-remitting clinical symptoms with each additional relapse.

Currently, the diagnosis of MS is based on clinical parameters, the number, size and location of lesions detected by MRI and spinal fluid diagnostics. Thus, it is often delayed due to heterogeneous symptoms and long recovery phases at the beginning of the disease preventing timely therapy initiation. Moreover, other neurologic diseases may mimic the symptoms in early phases (Rubegni et al., SPG2 mimicking multiple sclerosis in a family identified using next generation sequencing, J Neurol Sci. 375:198-202, Epub 2017 Jan 27). The currently used techniques, e.g. analysing cerebrospinal fluid obtained from a lumbar puncture, are, however, often painful for the patient and do not allow a specific diagnosis for MS only, nor for diagnosing an acute phase of MS.

Biomarkers that can accelerate the diagnosis of MS are an active research topic. Approaches include positron emission tomography addressing neuro-inflammation and astrocyte markers, genetic, immune-inflammatory, and oxidative stress markers, Vitamin D binding protein isoforms and apolipoprotein E in cerebrospinal fluid as well as plasma micro RNAs. Further blood-based biomarkers utilize metabolomic and proteomic markers or serum profiles of cytokines, chemokines and pro-apoptotic molecules (Del Boccio et al., Integration of metabolomics and proteomics in multiple sclerosis: From biomarkers discovery to personalized medicine, Proteomics. Clinical applications 10(4) (2016) 470-84; Hagman et al, Disease-associated inflammatory biomarker profiles in blood in different subtypes of multiple sclerosis: prospective clinical and MRI follow-up study, J Neuroimmunol 234(1-2) (2011) 141-7).

In particular, lipid metabolism has been suggested to be, among others, a major pathophysiological mechanism of multiple sclerosis (MS), up to a hypothesis that MS would be in fact a disease of lipid metabolism (Corthals, Multiple sclerosis is not a disease of the immune system, The Quarterly review of biology 86(4) (2011) 287-321). Among lipids, cholesterol and cholesterol turnover products have been associated with MS, whereas omega-3 lipids were protective by preserving the blood brain barrier. Recent investigations point at several further classes of lipids that are regulated in MS. Currently, a scientific focus centers on prostaglandins including hydroxyeicosatetraenoic acid, ceramides and lyosophosphatidic acids (Mattsson et al., Elevated cerebrospinal fluid levels of prostaglandin E2 and 15-(S)-hydroxyeicosatetraenoic acid in multiple sclerosis, J Intern Med 265(4) (2009) 459-64). The successful therapy of human MS with fingolimod, which antagonizes functions or sphingosine-1-phosphate (SIP) highlights the pathophysiologic relevance of bioactive lipids. In addition, recent research addressing ceramides in MS show that these lipids modify the course of experimental MS models (Barthelmes et al., Induction of Experimental Autoimmune Encephalomyelitis in Mice and Evaluation of the Disease-dependent Distribution of Immune Cells in Various Tissues, J Vis Exp 111, 2016). The benefits of cannabinoids for symptomatic control of MS-associated pain and muscle spasms and experimentally proven anti-neuro-inflammatory effects of cannabinoids further suggest a contribution of bioactive lipids for symptom control, resolution of inflammation and possibly remyelination (Corthals, Multiple sclerosis is not a disease of the immune system, The Quarterly review of biology 86(4) (2011) 287-321.).

Lötsch discloses the determination of serum concentrations of three main classes of lipid markers comprising eicosanoids, ceramides, and lyosophosphatidic acids in cohorts of MS patients and healthy subjects. It was shown that machine-learned data structures of lipid marker serum concentrations in multiple sclerosis patients differ from those in healthy subjects (Int. J. Mol. Sci. 2017, 18, 1217).

Although certain lipids such ceramides and lyosophosphatidic acids have been shown to be dysregulated in MS, their role in the course of disease is not completely clear.

Accordingly, multiple sclerosis is a chronic demyelinating disease that presents with variable signs and symptoms. This variability in the clinical presentation may result in misdiagnosis, unnecessary referrals and misleading information to the patients. Since MS is a clinically highly heterogeneous disease, specific lipid-based biomarkers or biomarker patterns that allow for identification of MS patients at high accuracy are urgently needed, but not yet available.

The technical problem underlying the present invention can be seen as the provision of means and methods for complying with the aforementioned needs. The technical problem is solved by

Accordingly, the present invention relates to a method for diagnosing multiple sclerosis (MS) in a subject suspected to suffer therefrom comprising the steps of:

a) determining the amount of at least one marker selected from the group consisting of LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) in a sample of said subject;
b) comparing the determined amount to a reference; and
c) diagnosing MS based on said comparison in said subject.

In a preferred embodiment of the method of the present invention, the amounts of at least three of the aforementioned biomarkers are determined in a sample from the subject. In an embodiment, the determined amounts are compared to reference amounts for the three markers, i.e. to three alternative reference amounts. In an alternative embodiment, a score is calculated based on the determined amounts and compared to a reference score.

In particular, the present invention thus relates to a method for diagnosing multiple sclerosis (MS) in a subject suspected to suffer therefrom comprising the steps of:

a) determining the amounts of at least three markers from a group of markers comprising LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) in a sample of said subject;
b) comparing the determined amounts to a reference or to references; and
c) diagnosing MS based on said comparison in said subject.

The references as referred to in step b) of the aforementioned method may be reference amounts for the individual marker(s). Further, the reference may be reference score which is calculated based on the amounts determined in step b).

The method as referred to in accordance with the present invention includes a method which essentially consists of the aforementioned steps or a method which includes further steps. However, it is to be understood that the method, in a preferred embodiment, is a method carried out in vivo, i.e. not practised on the human or animal body. The method, preferably, can be assisted by automation.

The term "diagnosing" as used herein refers to assessing whether a subject suffers from the disease MS, or not. As will be understood by those skilled in the art, such an assessment, although preferred to be, may usually not be correct for 100% of the investigated subjects. Preferably, a statistically significant portion of subjects can be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90%, at least 95%. The p-values are, preferably, 0.5, 0.3, 0.2, 0.1, 0.05, or 0.01. The actual diagnosis whether a subject suffers from MS, or not, may comprise further steps such as the confirmation of a diagnosis. Thus, the diagnosis of MS is understood as an aid in the diagnosis of MS. Accordingly, the term "diagnosing" in the context of the present invention also encompasses aiding the physician to assess whether a subject suffers from MS, or not.

The term "MS (multiple sclerosis)" as used herein relates to disease of the central nervous system (CNS) that causes prolonged and severe disability in a subject suffering therefrom. The pathogenesis of MS includes activation of encephalitogenic, i.e. autoimmune myelin-specific T cells outside the CNS, followed by an opening of the blood-brain barrier, T cell and macrophage infiltration, microglial activation, demyelination, and irreversible neuronal damage. There are four standardized subtype definitions of MS which are also encompassed by the term as used in accordance with the present invention: relapsing remitting, secondary progressive, primary progressive and progressive relapsing. The relapsing-remitting subtype is characterized by unpredictable relapses followed by periods of months to years of remission with no new signs of disease activity. Deficits suffered during attacks (active status) may either resolve or leave sequelae. This describes the initial course of 85 to 90% of subjects suffering from MS. In cases of so-called benign MS the deficits always resolve between active status. Secondary progressive MS describes those with initial relapsing-remitting MS, who then begin to have progressive neurological decline between acute attacks without any definite periods of remission. Occasional relapses and minor remissions may appear. The median time between disease onset and conversion from relapsing-remitting to secondary progressive MS is about 19 years. The primary progressive subtype describes about 10 to 15% of subjects who never have remission after their initial MS symptoms. It is characterized by progression of disability from onset, with no, or only occasional and minor, remissions and improvements. The age of onset for the primary progressive subtype is later than other subtypes. Progressive relapsing MS describes those subjects who, from onset, have a steady neurological decline but also suffer clear superimposed attacks. This is the least common of all subtypes.

Symptoms associated with MS include changes in sensation (hypoesthesia and paraesthesia), muscle weakness, muscle spasms, difficulty in moving, difficulties with coordination and balance (ataxia), problems in speech (dysarthria) or swallowing (dysphagia), visual problems (nystagmus, optic neuritis, or diplopia), fatigue, headache, acute or chronic pain, bladder and bowel difficulties. Cognitive impairment of varying degrees as well as emotional symptoms of depression or unstable mood may also occur as symptoms. The main clinical measure of disability progression and symptom severity is the Expanded Disability Status Scale (EDSS). Further symptoms of MS are well known in the art and are described in the standard text books of medicine, such as Stedman or Pschyrembl.

In an embodiment of the method of the present invention, the method allows for the diagnosis of early MS. Accordingly, the present invention relates to a method for the diagnosis of MS in an early stage of MS. The early stage of MS encompasses the time period of, preferably, two years, or even more preferably of one year after the onset of MS. Also preferably, the early stage of MS encompasses the time period from the first acute neurological event (attack) of MS until the second acute neurological event (attack) of MS. Accordingly, the subject to be tested is preferably is a subject who is exhibiting a first acute neurological event of MS. Alternatively, the subject to be tested is preferably a subject who has exhibiting a first acute neurological event of MS, but did not exhibit a further acute neurological event of MS. The expression "neurological event" is understood by the skilled person and preferably refers to a demyelinating event.

The term "biomarker" or "marker" as used herein refers to a molecular species which serves as an indicator for MS. Said molecular species shall be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition.

The biomarker(s) to be used in the context of the method of the present invention are shown in Table 2 of the Examples section. Accordingly, the biomarkers are LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide). The biomarkers are known by the skilled person:

15-Hydroxyeicosatetraenoic acid (also termed 15-HETE, 15(S)-HETE, and 15S-HETE) is an endogenous eicosanoid, i.e. a metabolite of arachidonic acid. The IUPAC name is (5*Z*,8*Z*,11*Z*,13*E*,15*S*)-15-Hydroxyicosa-5,8,11,13-tetraenoic acid.

Palmitoylethanolamide (PEA) is an endogenous fatty acid amide, belonging to the class of nuclear factor agonists. The IUPAC name is N-(2-Hydroxyethyl)hexadecanamide.

Oleoylethanolamine (OEA) is an endogenous peroxisome proliferator-activated receptor alpha (PPAR-α) agonist. The IUPAC name is (*Z*)-*N*-(2-Hydroxyethyl)octadec-9-enamide.

Biopterin is a pterin derivative which functions as endogenous enzyme cofactors in many species of animals and in some bacteria and fungi. The IUPAC name for the biomarker biopterin is 2-Amino-6-(1,2-dihydroxypropyl)-1*H*-pteridin-4-one.

C16 Dihydroceramide (d18:0/16:0) (herein also referred to as C16Sphinganine) is a sphingolipid. The IUPAC name is N-(1,3-Dihydroxy-2-octadecanyl)hexadecanamide.

C16 Glucosyl(β) Ceramide (d18:1/16:0) (herein also referred to as GluCer16) is a sphingolipid localized in the membrane. The IUPAC name is N-[(2S,3R,4E)-1-(β-D-Glucopyranosyloxy)-3-hydroxy-4-octadecen-2-yl]icosanamide.

C24:1 Lactosyl(β) Ceramide (d18:1/24:1/15Z) (herein also referred to as LacCer24:1) is a sphingolipid localized in the membrane. The IUPAC name is (15Z)-N-[(2S,3R,4E)-1-{[4-O-(β-D-Galactopyranosyl)-β-D-mannopyranosyl]oxy}-3-hydroxy-4-octadecen-2-yl]-15-tetracosenamide.

1-arachidonoyl-2-hydroxy-sn-glycero-3-phosphate (herein also referred to as LPA 20.4) is a metabolite of lysophosphatidyl choline. The IUPAC name is 1-(5Z,8Z,11Z,14Z-eicosatetraenoyl)-sn-glycero-3 -phosphate.

As set forth above, the amount at least one biomarker is preferably determined in accordance with the method of the present invention. However, more preferably, a group of biomarkers will be determined in order to strengthen specificity and/or sensitivity of the assessment. Such a group, preferably, comprises at least 2, at least 3, at least 4, at least 5, at least 6, at least 7, or all 8 of the aforementioned markers. In a preferred embodiment, the amounts of at least three biomarkers are determined in the method of the present invention. In another preferred embodiment, the amounts of all seven biomarkers are determined in the method of the present invention.

The order of preference of the aforementioned markers is as follows:

1. GluCerC16
2. HETE.15S
3. LPA20.4
4. Biopterin

5. OEA
6. LacCerC24.1
7. PEA
8. C16Sphinganin

In the studies underlying the present invention, the biomarker GluCerC16 gave the best results, followed by the biomarker HETE.15S, followed by the biomarker LPA20.4 etc. (see Examples section). Thus, the preferred biomarker is GluCerC16, followed by the biomarker HETE.15S, followed by the biomarker LPA20.4.

Accordingly, if more than one marker is determined, GluCerC16 is preferably one of the determined biomarkers. Further, it is envisaged that at least the amounts of GluCerC16 and HETE.15S are determined. Also, at least the amounts of GluCer16, LPA20:4 and HETE15S can be determined. Alternatively, at least the amounts of GluCer16, LPA20:4 and Biopterin can be determined. Further preferred combinations are as follows:

- at least the amounts of GluCer16, Biopterin and PEA, or

- at least the amount of GluCer16, Biopterin, LPA20:4 and HETE15S

In accordance with the present invention, it is in particular envisaged, to determine the amounts of at least one ethanolamide, of at least one hydroxyeicosatetranoic acid, of at least one sphingolipid, and of at least one lysophosphatidic acid (in step a of the method of the present invention). In addition to markers from these four lipid classes, the amount of biopterin can be determined.

The ethanolamide is preferably PEA or OEA, in particular OEA. The hydroxyeicosatetranoic acid is preferably HETE15S, the lysophosphatidic acid is preferably LPA20.4. The sphingolipid is preferably selected from the group consisting of GluCerC16, LacCerC24.1, and C16Sphinganine. In particular, the sphingolipid is GluCerC16.

In addition to the specific biomarkers recited in the specification, other biomarkers may be, preferably, determined as well in the methods of the present invention. Thus, the method of the present invention is preferably not restricted to the biomarkers mentioned herein.

The term "sample" as used herein refers to samples from body fluids, preferably, blood, plasma, serum, saliva, urine or cerebrospinal fluid, or samples derived, e.g., by biopsy, from cells, tissues or organs, in particular from the CNS including brain and spine. More preferably, the sample is a blood, plasma or serum sample, most preferably, a serum sample. Furthermore, it is envisaged that the sample is a plasma sample. Biological samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, dilution, fractioning, ultrafiltration, protein precipitation followed by filtration and purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

The term "subject" as used herein relates to animals and, preferably, to mammals. More preferably, the subject is a primate and, most preferably, a human. The subject, preferably, is suspected to suffer from MS, i.e. it may already show some or all of the symptoms associated with the disease.

The term "determining the amount" is well understood by the skilled person. As used herein the term preferably refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information (mass/charge ratio or quotient), as well as an intensity value being related to the abundance of the said biomarker (i.e. its amount) in the sample.

As discussed before, each biomarker comprised by a sample may be, preferably, determined in accordance with the present invention quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case were the precise amount of a biomarker can or shall not be determined. Quantitatively analysing a biomarker, thus, also includes what is sometimes referred to as semi-quantitative analysis of a biomarker. Accordingly, the term "amount" as used herein preferably encompasses the absolute amount of the biomarkers, the relative amount or concentration of the biomarkers.

In an embodiment of the method of the present invention, the amount(s) of the biomarker(s) that are determined in the sample from the subject are adjusted for age and gender of the subject. How to adjust the amount(s) is well known in the art. Preferably, the amounts are adjusted as indicated in the Examples section.

Moreover, determining as used in the method of the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado.

Most preferably, LC and/or GC are chromatographic techniques to be envisaged by the method of the present invention. Suitable devices for such determination of biomarkers are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Nissen 1995, Journal of Chromatography A, 703: 37-57, US 4,540,884 or US 5,397,894, the disclosure content of which is hereby incorporated by reference. As an alternative or in addition to mass spectrometry techniques, the following techniques may be used for compound determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

Moreover, the at least one biomarker can also be determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes. Specific antibodies, for instance, may be obtained using the biomarker as antigen by methods well known in the art. Antibodies as referred to herein include both polyclonal and monoclonal antibodies, as well as fragments thereof, such as Fv, Fab and $F(ab)_2$ fragments that are capable of binding the antigen or hapten.

As described above, said determining of the at least one biomarker can, preferably, comprise mass spectrometry (MS). Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis.

More preferably, said mass spectrometry is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

The reference in accordance with the present invention shall allow for the diagnosis of MS. The reference to be applied may be an individual reference for each of the biomarkers to be determined in the method of the present invention. Accordingly, the amount of each of the biomarkers is compared to a reference for each of the biomarkers.). Based on the comparison of the amounts of the biomarkers with the references, a diagnosis of MS, i.e. whether the subject as referred to herein suffers from MS, or not, can be established. The reference amount preferably refers to an amount which allows for allocation of a subject into either (i) the group of subjects suffering from MS or (ii) the group of subjects not suffering MS. A suitable reference amount may be determined from a reference sample to be analyzed together, i.e. simultaneously or subsequently, with the test sample.

The term "reference" preferably refers to values of characteristic features of each of the biomarker which can be correlated to the presence or absence of MS in the subject. Preferably, the reference is a reference amount. Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig MH. et al., Clin. Chem. 1993;39:561-577). The ROC graph is a plot of all the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1 - specificity for the complete range of thresholds suitable for making a distinction. On the y-axis is sensitivity, or the true-positive fraction, which is defined as the ratio of number of true-positive test results to the product of number of true-positive and number of false-negative test results. It is calculated solely from the affected subgroup. On the x-axis is the false-positive fraction, or 1 - specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/1 - specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis for a given event with a proper balance of sensitivity and specificity, respectively. Accordingly, the reference to be used for the method of the present invention, i.e. a threshold which allows to diagnose MS can be generated, preferably, by establishing a ROC for said cohort as described above and deriving a threshold amount therefrom. Dependent on a desired sensitivity and specificity for a diagnostic method, the ROC plot allows deriving a suitable threshold. It will be understood that an optimal sensitivity is desired for e.g. excluding a subject from suffering from MS (i.e. a rule out), whereas an optimal specificity is envisaged for a subject to be assessed as suffering from MS (i.e. a rule in). In a preferred embodiment the value for the characteristic feature can also be a calculated output such as score of a classification algorithm like "elastic net" as set forth elsewhere herein.

In a preferred embodiment of the method of the present invention, the reference or the references are derived from a subject or a group of subjects known to suffer from MS. In such cases, amounts for the biomarkers being essentially identical to the references are indicative for a subject suffering from MS.

In another preferred embodiment of the method of the present invention, the reference or the references are derived from a subject or a group of subjects known not to suffer from MS. In such cases, amounts for the biomarkers which are significantly different from the references are indicative for a subject suffering from MS.

In a further preferred embodiment, the term "reference amount" preferably refers to a predetermined value. Said predetermined value shall allow for diagnosing MS.

With respect to the biomarkers LacCerC24.1 and C16Sphinganine preferably the following applies: Preferably, an amount in the test sample being larger than the reference amount is indicative for the presence of MS while an amount being lower is indicative for the absence of MS.

With respect to the biomarkers GluCerC16, HETE.15S, LPA20.4, Biopterin, OEA and PEA preferably the following applies: Preferably, an amount in the test sample being lower than the reference amount is indicative for the presence of MS while an amount being larger is indicative for the absence of MS.

The same applies mutatis mutandis for a calculated reference, most preferably the average or median, for the relative or absolute amount of the at least one biomarker of a population of individuals comprising the subject to be investigated. The absolute or relative amounts of the at least one biomarker of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species. Preferably, the reference amounts or scores as referred to herein are derived from a control subject or group of control subjects, in particular a healthy subject or from a group of healthy subjects. A healthy subject as referred to herein does not suffer from MS. The reference amount applicable for a subject may vary depending on various physiological parameters, in particular on age and gender. Thus, it is envisaged that the test subject and the control subject(s) are age-matched and/or gender-matched. Alternatively, it is envisaged that the amount(s) of the biomarker(s) that are determined in the sample from the test subject are adjusted for age and gender of the subject (as described elsewhere herein in more detail).

The amounts of the test sample and the reference amounts are essentially identical, if the values for the characteristic features and, in the case of quantitative determination, the intensity values are essentially identical. Essentially identical means that the difference between two amounts is, preferably, not significant and shall be characterized in that the values for the intensity are within at least the interval between $1^{st}$ and $99^{th}$ percentile, $5^{th}$ and $95^{th}$ percentile, $10^{th}$ and $90^{th}$ percentile, $20^{th}$ and $80^{th}$ percentile, $30^{th}$ and $70^{th}$ percentile, $40^{th}$ and $60^{th}$ percentile of the reference value, preferably, the $50^{th}$, $60^{th}$, $70^{th}$, $80^{th}$, $90^{th}$ or $95^{th}$ percentile of the reference value. Statistical test for determining whether two amounts are essentially identical are well known in the art and are also described elsewhere herein.

An observed difference for two amounts, on the other hand, shall be statistically significant. A difference in the relative or absolute amount is, preferably, significant outside of the interval between $45^{th}$ and $55^{th}$ percentile, $40^{th}$ and $60^{th}$ percentile, $30^{th}$ and $70^{th}$ percentile, $20^{th}$ and $80^{th}$ percentile, $10^{th}$ and $90^{th}$ percentile, $5^{th}$ and $95^{th}$ percentile, $1^{st}$ and $99^{th}$ percentile of the reference value. Preferred changes and fold-regulations are described in the accompanying Tables as well as in the Examples.

Preferably, the reference, i.e. values for at least one characteristic features of the at least one biomarker, will be stored in a suitable data storage medium such as a database and are, thus, also available for future assessments.

In the context of step b) of the present invention, the amounts of the biomarkers as referred to in step a) of the methods of the present invention shall be compared to a reference or references. Thereby, the presence or absence of MS is diagnosed. As set forth above, references for the individual determined biomarkers, i.e. references for each of biomarkers as referred to in step a) are applied. However, it is also envisaged to calculate a score (in particular a single score) based on the amounts of the at least three biomarkers as referred to in step a) of the method of the present invention, i.e. a single score, and to compare this score to a reference score. The calculated score combines information on the amounts of the biomarkers as set forth above. The score can be regarded as a classifier parameter for diagnosing MS. In an embodiment, the reference score is preferably a value, in particular a cut-off value which allows for differentiating between the presence of MS and the absence of MS in the subject to be tested.

Thus, in a preferred embodiment of the present invention, the comparison of the amounts of the biomarkers to a reference as set forth in step b) of the method of the present invention encompasses step b1) of calculating a score based on the determined amounts of the biomarkers as referred to in step a), and step b2) of comparing the, thus, calculated score to a reference score.

The term "comparing" refers to determining whether the determined amount of a biomarker is essentially identical to a reference or differs therefrom. Preferably, a biomarker is deemed to differ from a reference if the observed difference is statistically significant which can be determined by statistical techniques referred to elsewhere in this description. If the difference is not statistically significant, the biomarker amount and the reference amount are essentially identical. Based on the comparison referred to above, a subject can be assessed to suffer from the disease, or not. The comparison is, preferably, assisted by automation. For example, a suitable computer program comprising algorithms for the comparison of two different data sets (e.g., data sets comprising the values of the characteristic feature(s)) may be used. Such computer programs and algorithm are well known in the art. Notwithstanding the above, a comparison can also be carried out manually.

For the specific biomarkers referred to in this specification, preferred reference amounts are indicated in Table 2 in the Examples below. Further, the kind of regulation (i.e. "up"- or "down"-regulation resulting in a higher or lower relative and/or absolute amount) is indicated in Table 2 as well. If it is indicated in said table that a given biomarker is "up-regulated" (indicated by an ">") in a subject, the relative and/or absolute amount is preferably increased with respect to the reference amount in a subject suffering from MS and decreased with respect to the reference amount in a subject not suffering from MS. This is the case for the biomarkers LacCerC24.1 and C16Sphinganine. If it is indicated in said table that a given biomarker is "down-regulated" (indicated by an "<") in a subject, the relative and/or absolute amount is preferably decreased with respect to the reference amount in a subject suffering from MS and increased with respect to the reference amount in a subject not suffering from MS. This is the case for the biomarkers GluCerC16, HETE.15S, LPA20.4, Biopterin, OEA and PEA.

In a preferred embodiment of the method of the present invention, the amounts of all eight biomarkers as referred to in step a) are determined and compared to references. Preferably, a subject suffers from MS is at least three of the following eight criteria a met:

1. the amount of GluCerC16 in the sample from the subject is lower than the reference amount
2. the amount of HETE.15S in the sample from the subject is lower than the reference amount
3. the amount of LPA20.4 in the sample from the subject is lower than the reference amount
4. the amount of Biopterin in the sample from the subject is lower than the reference amount
5. the amount of OEA in the sample from the subject is lower than the reference amount
6. the amount of LacCerC24.1 in the sample from the subject is larger than the reference amount
7. the amount of PEA in the sample from the subject is lower than the reference amount
8. the amount of C16Sphinganin in the sample from the subject is larger than the reference amount

The criteria above in particular apply to serum samples.

Advantageously, it has been found in the study underlying the present invention that the amounts of the specific biomarkers referred to above are indicators for MS. Accordingly, the at least one biomarker, in particular the at least three biomarkers as specified above in a sample can, in principle, be used for assessing whether a subject suffers from MS. This is particularly helpful for an efficient diagnosis of the disease as well as for improving of the management of MS as well as an efficient monitoring of patients.

In a preferred embodiment of the method of the present invention, the method further comprises recommending or initiating a therapy of MS in a subject who has been diagnosed to suffer from MS by the method of the present invention. Preferably, said therapy is a drug-based therapy of MS. More preferably, said therapy comprises administration of at least one drug selected from the group consisting of: Interferon Betala, Interferon Beta 1b, Azathioprin, Cyclophosphamide, Glatiramer Acetate, Immunglobuline Methotrexat, Mitoxantrone, Natalizumab, Teriflunomid, Statins, Daclizumab, Alemtuzumab, RitximabCladribine, Fumarate, and Laquinimod.

Furthermore, the present invention relates to the in vitro use of at least one marker selected from the group consisting of LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) for diagnosing MS in a subject.

In particular, the present invention relates to the in vitro use of at least three markers from a group of markers comprising LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) for diagnosing MS in a subject.

FIGURES

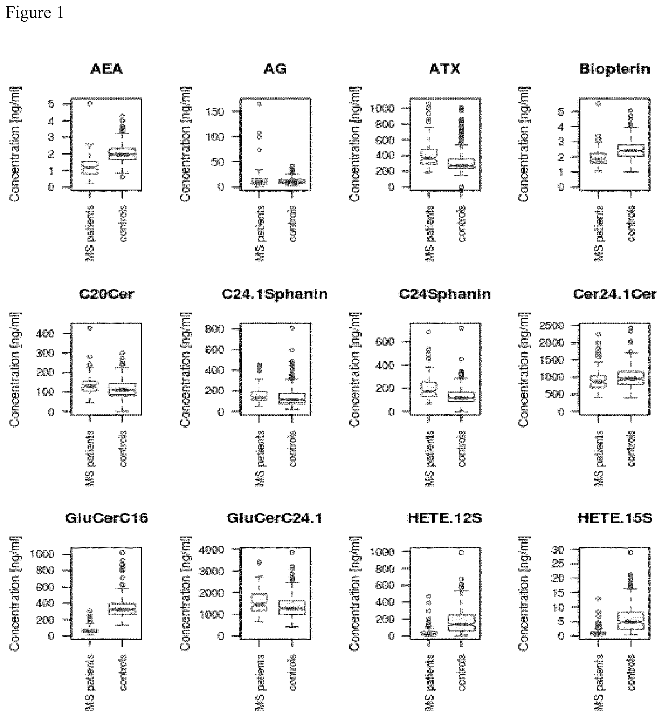

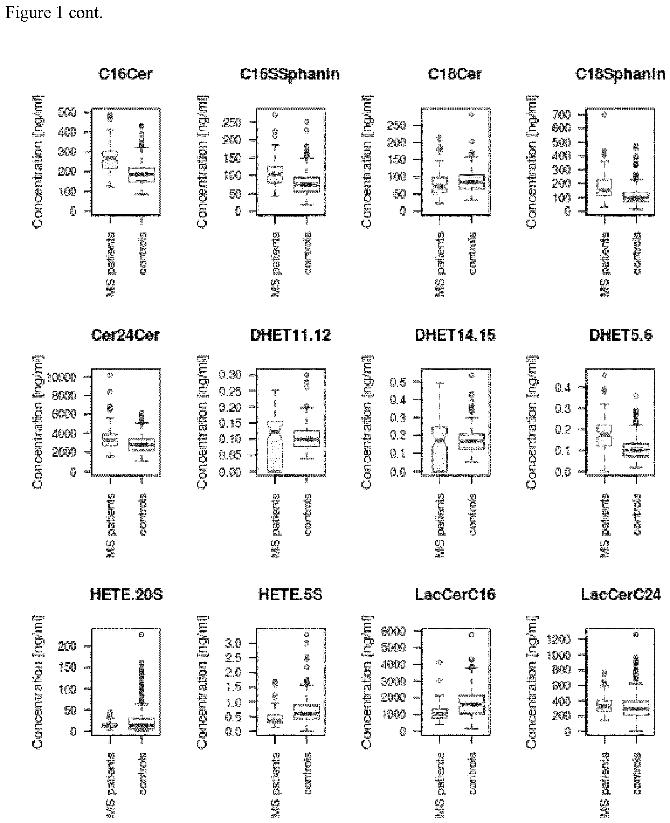

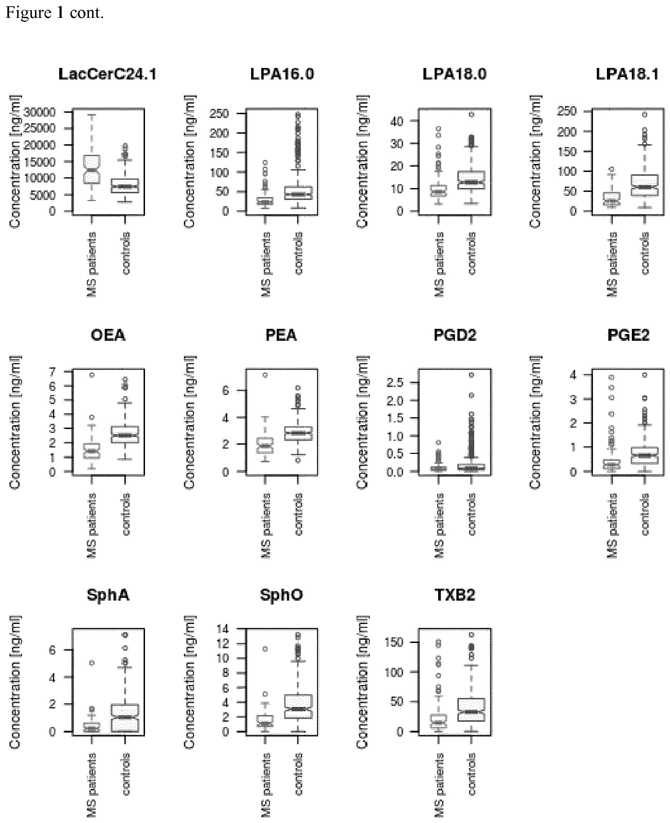

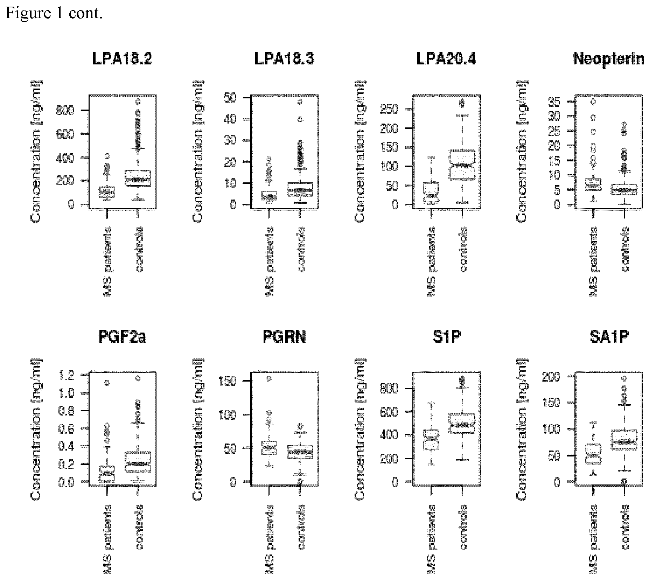

**Figure 1:** Serum concentrations of d = 43 lipid mediators (raw data). The data are shown in alphabetical order of marker names and for each marker, separately for group membership to the multiple sclerosis patients (left boxes, red) or the healthy subjects (right boxes, green). The widths of the boxes are proportional to the respective numbers of subjects per group. The quartiles and medians (solid horizontal line within the box) are used to construct a "box and whisker" plot. The whiskers add 1.5 times the interquartile range (IQR) to the 75th percentile or subtract 1.5 times the IQR from the 25th percentile and are expected to include 99.3% of the data if normally distributed. The notches indicate the confidence interval around the median based on median $\pm 1.57 \cdot IQR/n^{0.5}$. The figure has been created using the R software package (version 3.4.0 for Linux).

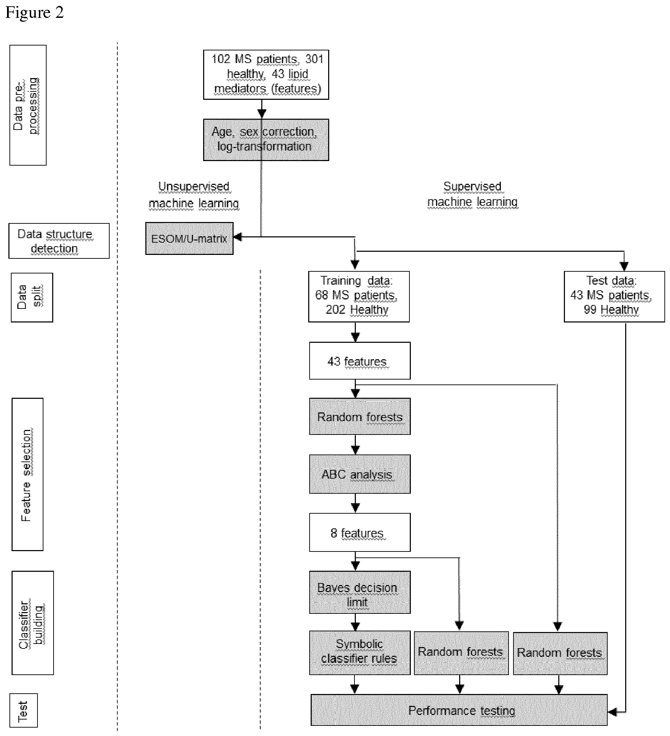

**Figure 2:** Flow chart of the data analysis. The figure provides an overview on the applied machine-learning approach in three main steps (indicated at the left side: data preprocessing, feature selection and classifier building including testing). The white frames show the variable flow, along with group size information. The grey frames depict the bioinformatics operations applied on the variables. During feature selection, the number of variables qualifying as components of a diagnostic tool respectively classifier was stepwise reduced, forwarding to the next analytical step only those features that had passed the criteria of the actual selection procedure.

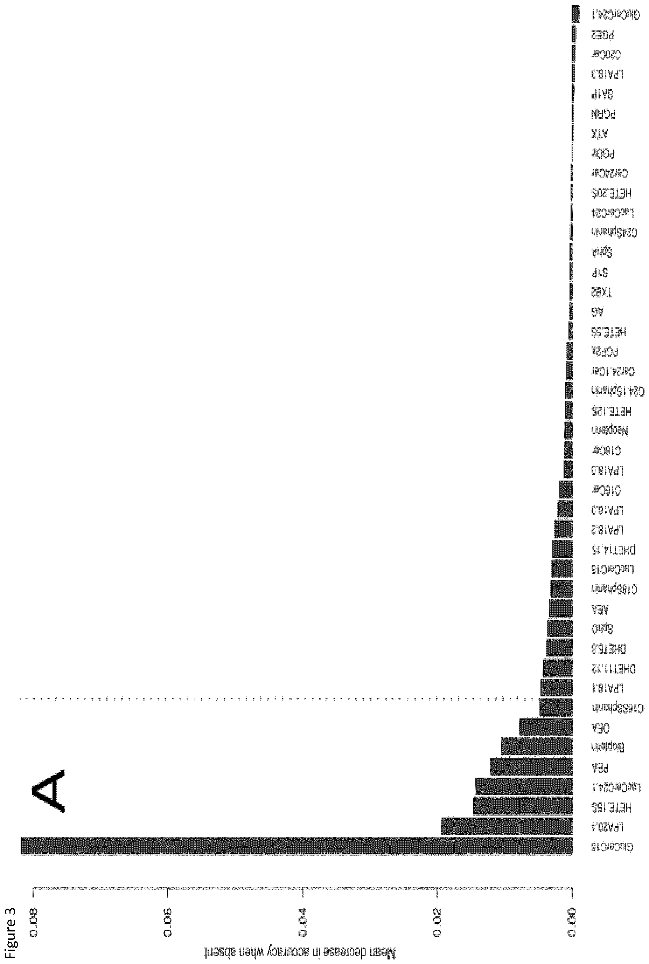

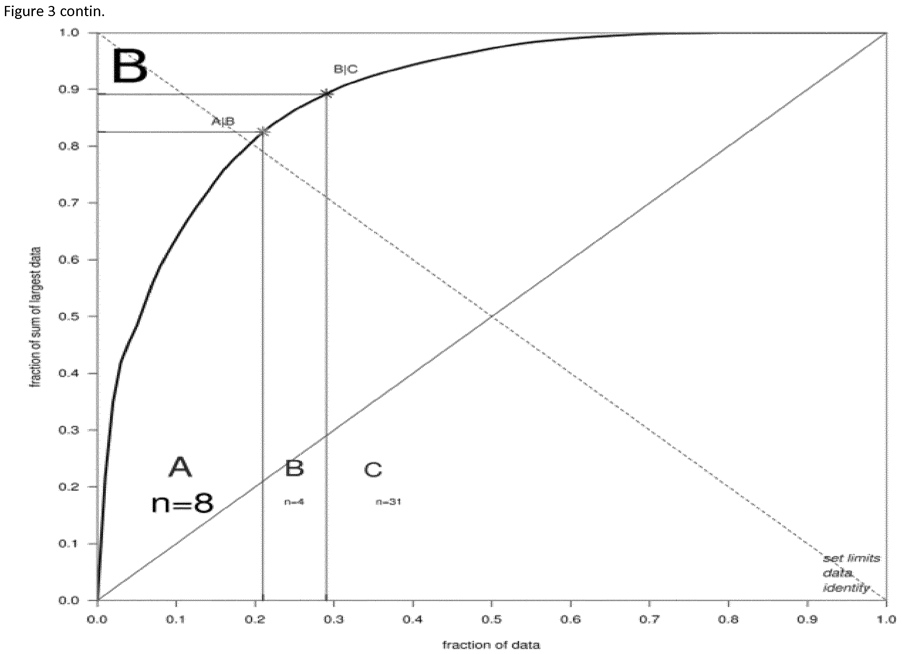

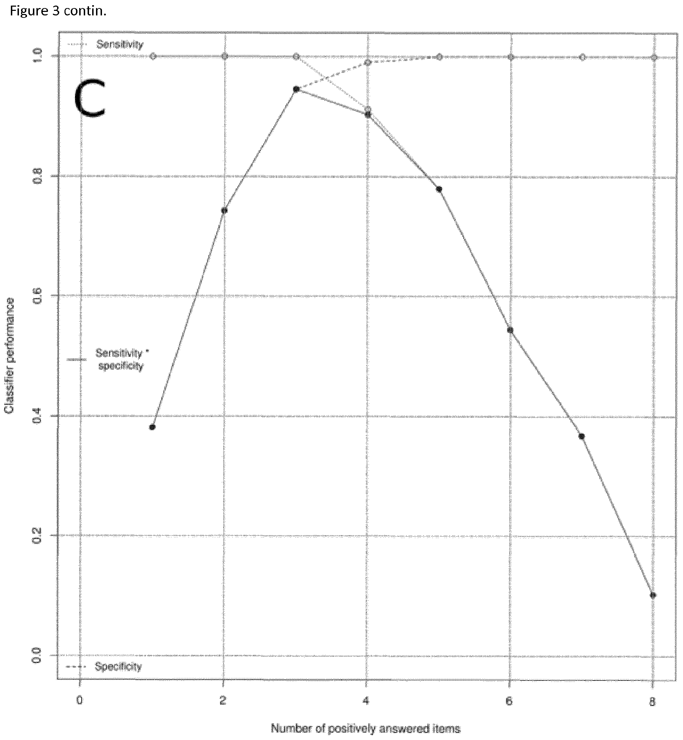

**Figure 3:** Feature selection and classifier performance. A: Feature selection using random forest machine learning and ABC analysis. A first step of feature selection was analyzing the mean decrease of accuracy over all cross-validated predictions, i.e., the change in the number of observations that are incorrectly classified, when the respective variable is removed. The bar plot shows the decrease in accuracy, as positive values, in descending numerical order. The plot depicts one example out of 1,000 runs and therefore, the order differs from the overall importance order of the parameters as given in Table 2. B: Subsequently, the mean decrease of accuracy associated with each variable was submitted to ABC analysis, which is an item selection procedure aiming at identification of most profitable items from a larger list of items. The ABC plot (blue line) shows the cumulative distribution function of the mean decreases in accuracy, along with the identity distribution, xi = constant (magenta line, i.e., each feature contributes similarly to the classification accuracy. The plots shows the same example run as shown in A. C: Plot of the classifier building procedure calculating test performance parameters for every possible sum of positive responses to the rules in Table 2. The solid line shows the product of sensitivity and specificity for different item sums. The dashed lines show the respective test sensitivity and specificity for every sum of positive items. The classification rule tested was "if the sum of the rules that applied was smaller than the actual iteration number then the patient belongs to the "healthy subject" group, else to the "multiple sclerosis" group. The analysis was done iteratively with item sums increasing by a value of one between each iteration. The figure has been created using the R software package (version 3.4.0 for Linux; http://CRAN.R-project.org/). In particular, the ABC analysis was performed and plotted using our R package "ABCanalysis".

All references cited herein are herewith incorporated by reference with respect to their disclosure content in general or with respect to the specific disclosure contents indicated above.

The invention will now be illustrated by the following Examples which are not intended to restrict or limit the scope of this invention.

EXAMPLES

**Example 1: Subjects and study design**

The study followed the Declaration of Helsinki and was approved by the Ethics Committee of the Medical Faculty of the Goethe - University Frankfurt am Main, Germany. Informed written consent was obtained from all subjects. Employing a parallel group design, patients with multiple sclerosis (n = 102, aged 18.2 - 62.8 years, 31 men) and healthy controls (n = 301, aged 18 - 53.2 years, 118 men) were consecutively recruited from outpatients and inpatients of the Department of Neurology (patients) and from students and staff members of the hospital (controls) who routinely reported at the institutional occupational health service. Patients and healthy subjects showed a similar sex distribution ($\chi$2-test : $\chi$2 = 2.1738, degrees of freedom, df = 1, p = 0.1404) but were differently aged (Wilcoxon signed rank test: W = 25,834, p < 2.2 · 10-16), which was taken care of during data preprocessing (see respective section below). Data and blood collection of MS patients was part of the local bio-banking project (Neurological Department of the Goethe University, Frankfurt). Inclusion criteria were age $\geq$ 18 years, for patients a clinically verified diagnosis of multiple sclerosis based on McDonald criteria and for controls no current medical condition queried by medial interview, and no drug intake for at least one week except contraceptives, vitamins and L-Thyroxin. Demographic data including time since diagnosis, Expanded Disability Status Scale (EDSS) (Kurtzke, Rating neurologic impairment in multiple sclerosis: an expanded disability status scale (EDSS), Neurology 33(11) (1983) 1444-52) and current disease modifying medication are summarized in Table 1.

**Table 1:** Demographic parameters of the MS patients, disease characteristics and medications. EDSS: Expanded Disability Status Scale, RRMS: Relapsing Remitting MS, PPMS: Primary Progressive MS, SPMS: Secondary Progressive MS

| | MS 1st course | | | | | | RRMS no relapse | | | | | |
|---|---|---|---|---|---|---|---|---|---|---|---|---|
| | Mean | SD | n | Mean | SD | n | Mean | SD | n | Mean | SD | n |
| Sex | m | | | f | | | m | | | f | | |
| Age | 38.2 | 7.5 | 8 | 31.6 | 9.1 | 12 | 35.5 | 9.4 | 16 | 34.3 | 8.9 | 39 |
| Disease years | 0.03 | 0.04 | | 0.9 | 2.9 | | 7.5 | 6.1 | | 7.4 | 5.9 | |
| EDSS | 0.6 | 1.0 | | 0.8 | 1.1 | | 3.1 | 1.7 | | 2.3 | 1.6 | |
| Medication | | | | | | | | | | | | |
| None | | | 6 | | | 11 | | | 3 | | | 6 |
| β-Interferon | | | 0 | | | 0 | | | 6 | | | 11 |
| Fingolimod | | | 0 | | | 0 | | | 3 | | | 5 |
| Natalizumab | | | 0 | | | 0 | | | 4 | | | 15 |
| Other | | | 2 | | | 1 | | | 0 | | | 2 |
| | RRMS acute relapse | | | | | | SPMS or PPMS | | | | | |
| | Mean | SD | n | Mean | SD | n | Mean | SD | n | Mean | SD | n |
| Sex | m | | | f | | | m | | | f | | |
| Age | 33.8 | 7.0 | 4 | 37.2 | 10.9 | 18 | 43.6 | 18.1 | 3 | 49.2 | 11.0 | 2 |
| Disease years | 2.1 | 1.8 | | 6.7 | 5.3 | | 5.7 | 4.7 | | 1.0 | 1.3 | |
| EDSS | 1.63 | 1.2 | | 2.0 | 1.9 | | 4.0 | 2.8 | | 5.0 | | |
| Medication | | | | | | | | | | | | |
| None | | | 1 | | | 10 | | | 1 | | | 1 |
| β-Interferon | | | 2 | | | 5 | | | 0 | | | 1 |
| Fingolimod | | | 0 | | | 1 | | | 1 | | | 0 |
| Natalizumab | | | 0 | | | 0 | | | 0 | | | 0 |
| Other | | | 1 | | | 2 | | | 1 | | | 0 |

Lipid mediator serum concentration analysis

From each subject, a venous blood sample (9 ml) was collected into a serum tube and centrifuged at 3,000 rpm for 10 min. Serum was separated and frozen at -80 °C until assay. A total of d = 43 different lipid mediators (Figure 1) was analyzed from the serum samples. Sphingolipids were extracted as follows. 50 μl sample were mixed with 150 μl water, 150 μl extraction buffer (citric acid 30 mM, disodium hydrogen phosphate 40 mM) and 20 μl of the internal standard solution containing sphingosine-d7, sphinganine-d7 (200 ng/ml each), sphingosine-1-phosphate-d7, C17:0 Cer, C16:0 Cer-d31, C18:0 Cer-d3, C17:0 LacCer, C18:0 DHC-d3, C16:0 LacCer-d3, C18:0 GluCer-d5 (all avanti polar lipids, Alabaster, USA) and C24:0 Cer-d4 (Chiroblock GmbH, Bitterfeld-Wolfen) (400 ng/ml each). The mixture was extracted once with 1000 μl methanol/chloroform/hydrochloric acid (15:83:2, v/v/v). The lower organic phase was evaporated at 45 °C under a gentle stream of nitrogen and reconstituted in 200 μl of tetrahydrofuran/water (9:1, v/v) with 0.2% formic acid and 10 mM ammonium formate. Afterwards, amounts of sphingolipids were analyzed by LC-MS/MS. LPAs were extracted as follows. 2 cycles of liquid-liquid extraction of LPAs were done with 1-buthanol after adding the internal standard LPA17:0 (50 μl sample, 20 μl internal standard in 500 μl 1-butanol). The combined organic phases were dried under a gentle stream of nitrogen at 45 °C and subsequently re-dissolved in 200 μl methanol. The selection

included ceramides (Cer16:0, Cer18:0, Cer18:1, Cer20:0, Cer24:0, Cer24:1, GluCerC16:0, GluCerC24:1, LacCerC16:0, LacCerC24:0, LacCerC24:0), lyosophosphatidic acids (LPA16:0, LPA18:0, LPA18:1, LPA18:2, LPA18:3, LPA20:4)), sphingolipids (sphinganine, sphingosine, SIP, SA1P C16Sphinganine, C18Sphinganine, C24Sphinganine, C24:1Sphinganine), prostaglandins (PGD2, PGF1a, PGE2, TXB2), dihydroxyeicosatrienoic acids (DHET5,6, DHET11,12, DHET14,15), hydroxyeicosatetranoic acids (HETE 5S, HETE_12S, HETE_15S, HETE_20S), endocannabinoids (AEA, OEA, PEA, 2-AG) and pterins (biopterin, neopterin). Pterins were included because tetrahydrobiopterin regulates the metabolism of multiple bioactive lipids as a coenzyme of alkylglycerolmonooxygenase (AGMO).

Serum concentration analyses were performed using liquid chromatography-electrospray ionization-tandem mass spectrometry (LC-ESI-MS/MS) as described previously (Zschiebsch et al.,, Tetrahydrobiopterin attenuates DSS-evoked colitis in mice by rebalancing redox and lipid signaling, J Crohns Colitis 29 2016) In brief, eicosanoids (DHET11.12, DHET14.15, DHET5.6, HETE.12S, HETE.15S, HETE.20S, HETE.5S, PGD2, PGE2, PGF2a, TXB2; PGD2 = prostaglandin D2, PGE2 = prostaglandin E2, PGF2a = prostaglandin F2a, TXB2 = thromboxane, DHET = dihydroxyeicosatrienoic acid, HETE = hydroxyeicosatetraenoic acid) were assayed in two different LC-MS/MS runs. Prostanoids were separated using a Synergi Hydro column (150x2 mm, 4 $\mu$m, Phenomenex, Aschaffenburg, Germany) and the analysis of HETE/DHET was done using a Gemini NX column (150 x 2 mm, 5 $\mu$m, Phenomenex). In both cases, quantification was performed using a triple quadrupole mass spectrometer QTRAP 5500 (Sciex, Darmstadt, Germany) equipped with a Turbo-V-source operating in negative ESI mode. Ceramides (C16Cer, C18Cer, C20Cer, Cer24Cer, Cer24.1Cer, GluCerC16, GluCerC24.1, LacCerC16, LacCerC24, LacCerC24.1; Cer = ceramide, GluCer = glucosylceramide, LacCer = lactosylceramide) were analyzed using a Luna C18 column (150x2 mm ID, 5 $\mu$m particle size, Phenomenex) coupled to an API 4000 mass spectrometer equipped with an APCI (Atmospheric Pressure Chemical Ionization) ion source operating in positive mode (Sciex). The analysis of lysophosphatidic acids (LPA16.0, LPA18.0, LPA18.1, LPA18.2, LPA18.3, LPA20.4) was performed using a Mercury Luna C18 column (20x2 mm, 3 $\mu$m, Phenomenex) coupled to a triple quadrupole mass spectrometer (QTRAP 5500) operating in negative ESI mode. In all cases, the analytes were extracted using liquid-liquid-extraction prior to LC-MS/MS-analysis. Sample volumes were 200 $\mu$L each for prostanoids and DHET/HETE, 20 $\mu$L for ceramides and 50 $\mu$L for LPA and endocannabinoids.

For all analytes, the concentrations of the calibration standards, quality controls and samples were evaluated by Analyst software 1.6 and MultiQuant Software 3.0 (Sciex) using the internal standard method (isotope-dilution mass spectrometry). Calibration curves were calculated by linear regression with 1/x weighting for ceramides and LPA and by quadratic regression with $1/x^2$ weighting for eicosanoids.

**Example 2: Data analysis**

Data were analyzed using the R software package (version 3.4.0 for Linux on an Intel Xeon® computer running on Ubuntu Linux 16.04.2 64-bit. The acquired parameters, in the following called "features", included d = 43 lipid mediators assayed from the participants' venous blood serum (Figure 2). The analysis was performed in four main steps (Figure 1) comprising (i) data preprocessing, (ii) identification of lipid marker patterns and relation of this patterns to the clinical diagnosis, (iii) classifier building including features election and performance testing and (iv) biomedical interpretation of the identified subset of lipid markers sufficient to diagnose MS. Unsupervised machine learning implemented as implemented emergent self-organizing feature maps (ESOM) combined with the U-matrix, which is a topology-preserving artificial neuronal network (Kohonen, Self-organized formation of topologically correct feature maps, Biol Cybernet 43 (1982) 59-69) shown to provide unbiased structure identification in biomedical data was used to establish firstly whether the set of d = 43 lipid markers provides a suitable basis to separate MS patients from healthy subjects. In this approach, the goal is find "interesting" structures in an unlabeled data set of $D = \{(x_i)|x_i \in X, i = 1 ... n\}$, composed of values

$x_i \in X \subset \mathcal{R}^d$ comprising the d features, respectively serum concentrations of lipid markers. In the case that the identified distance and density based data structures coincided with the known diagnostic groups, i.e., MS or healthy, the d = 43 lipid markers were regarded as providing information suitable to separate patients from controls. In that case, supervised machine-leaning was used subsequently, implemented as random forests combined with Bayesian statistics, to develop a biomarker from the available features. Here, a classifier was desired that (i) was able to provide a correct diagnosis of the presence or absence of multiple sclerosis while (ii) the individual contributors to the complex marker remained accessible for functional interpretation. In the supervised machine learning approach, the goal is to learn a mapping from inputs x to output y, given a labeled set of input-output pairs $D = \{(x_i,y_i)|x_i \in X, y_i \in Y, i = 1...n\}$, composed of pairs of Boolean values $y_i \in Y = B$ comprising the diagnoses of either "multiple sclerosis" (1) or "healthy" (0) and values

$x_i \in X \subset \mathcal{R}^d$ comprising the d features, respectively serum concentrations of lipid markers, possibly predicting these diagnoses. The classifier is firstly trained in a labeled data set and subsequently applied to novel data where its diagnostic performance is assessed. To this end, the original data set was randomly split into a 2/3 sized training data set and a 1/3 sized test data set that both contained the two study groups in size proportional counts. The data analysis is described in detailed in the following.

Data preprocessing

A single outlier in the DHET5.6 serum concentrations was eliminated on the basis of a significant Grubbs test ($G = 19.624$, $U = 0.03966$, $p < 2.2 \cdot 10^{-16}$). Subsequently, data was preprocessed to correct for age and sex effects (significant differences in correlation analyses or Wilcoxon signed rank tests in several parameters, details not shown) based on linear regression or median differences, respectively. Following this correction, the statistically significant effects of age and sex were eliminated (age: Spearman's $\rho$ non-significant in all markers; sex: Wilcoxon signed rank tests non-significant in all markers. Quantile-quantile plots suggested a zero invariant log-transformation to LogConcentration = ln(Concentration + 1), which is in line with general advices for transformations of blood-concentration data.

Detection of data structures and their relation to MS diagnosis

The data space $D = \{(x_i)|x_i \in X, i = 1 \dots n\}$ comprising the serum concentrations of d = 43 lipid markers acquired from n = 403 subjects was explored for structures that possibly overlapped with the known data labeling respectively group structure of MS patients or controls. This was performed by means of unsupervised machine learning implemented as emergent self-organizing feature maps (ESOM) that were combined with the U-matrix. Specifically, ESOM are based on a topology-preserving projection of high-dimensional data points $x_i \in R^D$ onto a two dimensional self-organizing network consisting of a grid of neurons. This approach represents a topology preserving mapping of high-dimensional data points onto a two dimensional grid of neurons which was therefore favored to more common clustering algorithms such as k-means, Ward, complete- and average linkage that in contrast to the ESOM/U-matrix method are prone to detect false structures in the data. The neural network consisted of a two-dimensional toroid grid of so-called neurons with 50 rows and 80 columns (n = 4,000 units, for SOM size determination). Each neuron holds, in addition to a position vector on the two-dimensional grid, a further vector carrying "weights" of the same dimensions as the input dimensions. The weights were initially randomly drawn from the range of the data variables and subsequently adapted to the data during the learning phase that used 20 epochs respectively sweeps through the data.
Following training of the neural network, an ESOM was obtained that represented the subjects on a two-dimensional toroid map as the localizations of their respective "best matching units" (BMU), which are neurons on the grid that after ESOM learning carried the vector that was most similar to a subjects' data vector. On top of this grid, the distances between data points are calculated with the U-matrix. Every value (height) in the U-matrix depicts the average high-dimensional distance of a prototype to all immediate neighboring prototypes regarding a grid position. The corresponding visualization technique is a topographical map with hypsometric colors facilitating the recognition of distance and density based structures. Large "heights" in brown and white colors represent large distances between data points (subjects) separating "valleys" in green and blue colors that represent data points that are similar. These calculations were done using our R library "ESOM" (M. Thrun, F. Lerch, Marburg, Germany

Classifier creation

The biomarker, respectively classifier, was created using supervised machine learning implemented as random forests combined with Bayesian statistics. To obtain a a classifier was desired that accessible for functional interpretation and to avoid that unnecessary laboratory analytics were required, a feature selection step was included in the analysis with the aim to identify, among the d = 43 lipid markers, only a subset on which a sensitive diagnose can be based.

Feature selection

Feature selection for classifier development was started with a random forest analysis, which creates sets of different, uncorrelated, and often very simple decision trees with conditions on variables (features) as vertices and classes as leaves. Each tree in the random forest votes for a class and the final classification assigned to a data point follows the majority of these class votes. In the present analysis, 500 decision trees containing up to six randomly drawn features were calculated on data with equal group sizes randomly drawn from the training data set. The number of trees was heuristically based on visual analysis of the relationship between the number of decision trees and the classification accuracy. This indicated no improvement beyond 100 trees, from which 500 trees were considered to provide robust results. To establish that this heuristics did not affect the results of classifier creating, different numbers such as 100 or 1,000 were also tested, which always led to the selection of the same lipid markers as members of the final biomarker.
From these trials, features respectively lipid markers were chosen to be included into the final classifier based on the mean decrease in classification accuracy when the respective feature was excluded from random forest building.

The amount of this decrease indicated the importance of the particular feature. This procedure was repeated on 1,000 different training and test data sets randomly drawn from the original training data set. Following the concept of a nested cross-validation analysis, the random forest analysis was performed using randomly drawn subsamples from the actual training sample and the forest was applied to the actual test sample. These calculations were done using the R library "randomForest".

After each random forest analysis, the above-obtained values of the mean decrease in tree classification accuracy - when the feature was excluded from random forest analysis - were subsequently submitted to calculated ABC analysis. This is a categorization technique for the selection of a most important subset among a larger set of items. It was chosen since it fitted to the basic requirements of feature selection using filtering techniques, i.e. it does easily scale to very high-dimensional datasets, is computationally simple and fast, and independent of the classification algorithm. ABC analysis aims at dividing a set of data into three disjoint subsets called "A", "B" and "C". Subset "A" can be regarded as containing the most profitable features and were therefore chosen for classifier establishment. For each of the 1,000 runs, the size and members of ABC set "A" were retained. The final size of the feature set was equal to the most frequent size of set "A" in the 1,000 runs, and the final members of the feature set were chosen in decreasing order of their appearances in ABC set "A" among the 1,000 runs. These calculations were done using our R package "ABCanalysis".

Before creating the final classifier, each of the selected features was judged by a topical expert who verified that no major evidence exists in the published medical literature that the feature is discouraged to be included in a diagnostic tool for multiple sclerosis. An exclusion criterion would be that the parameter is known to be regulated or used as a marker for non-MS CNS diseases such as pathogen-caused or autoimmune-mediated inflammatory CNS diseases, neuroborreliosis, brain tumor, spinal ischemia, sarcoidosis, vasculitis, acute disseminated encephalopathy or leukodystrophy.

Classifier building

The lipid markers having passed the above feature selection were subsequently used to generate the classifier. To this end, for each feature f a classification rule $\psi(f) \rightarrow Y$ was used to assign a class label to the data on the basis of the observation of its feature vector by partitioning the feature space in two decision regions $F_1$, $F_2$ (yes or no for the diagnosis "multiple sclerosis", respectively) For each feature f the probability density function (PDF) of the log-transformed lipid mediator concentrations was modeled by optimizing a Gaussian mixture model (GMM) to the distribution. The GMM was given by the equation

$$p(x_f) = \sum_{i=0}^{M} p_i(x_f) = \sum_{i=0}^{M} w_i N(x_f | Mean_i, SD_i) = \sum_{i=1}^{M} w_i \cdot \frac{1}{\sqrt{2 \cdot \pi \cdot SD_i}} \cdot e^{-\frac{(x_f - Mean_i)^2}{2 SD_i^2}}$$

where

$N(x_f | Mean_i, SD_i)$ denotes Gaussian probability densities (component, mode) with means, $Mean_i$ and standard deviations, $SD_i$. The $w_i$ are the mixture weights indicating the relative contribution of each component Gaussian to the overall distribution, which add up to a value of 1. M denotes the number of components in the mixture. GMM fitting and optimization was performed using the R package "AdaptGauss" (https://cran.r-project.org/package=AdaptGauss).

The Bayesian decision rule

$$\psi(f) = 1, if\ p_i(f) > p_j(f)\ and\ mean(f|\text{multiple sclerosis}) > mean(f|\text{healthy}), else\ 0$$

was used to assign a decision score to each feature f. This identified specific "yes/no" thresholds for each of the features.

The final classifier was created by identifying a classification classification rule $\psi_d(t): \mathcal{R}^d \rightarrow Y$, $\psi_d(t) = SF > t$ that assigned a class label to the data based on the sum of decision scores SF of the features, where t is a threshold on this sum. All possible values of the threshold t from 1 to |{ABC set A}| were iteratively assessed with respect to the product of sensitivity and specificity. This identified an optimum threshold for patient classification. In this classifier, the relevant lipid mediator concentrations were represented by rules that are comprehensible for biomedical experts, which according to artificial intelligence concepts defines a symbolic classifier.

An alternative sub-symbolic classifier was generated by means of random forest machine learning using the same |{ABC set A}| lipid markers as used in the symbolic classifier. In this classifier, the mediator concentrations are represented by the many trees of the forest. This eluded direct interpretation by biomedical experts in the sense that the classification can be obtained but the details about the lipid mediators triggering the decision are hidden. Finally, a further random forest based classifier was created using the complete set of d = 43 lipid markers, with a complete nested cross validated approach and additional randomization of the number of features included in each tree and the number of trees built in each run. This procedure was implemented in 10,000 runs on resampled data.

Classifier performance testing

The performances of all classifiers were assessed on the test data set drawn at the start of the data analysis and comprised the calculation of standard measures of test performance (sensitivity, specificity, balanced accuracy). The 95 % confidence intervals of the performance test parameters were obtained as the 2.5th and 97.5th percentiles of the results of 1,000 runs on Bootstrap resampled data.

**Example 3: Identification of lipid mediator serum concentration patterns by unsupervised machine-learning**

Serum concentrations of d = 43 lipid markers were available from 102 patients with multiple sclerosis and 301 healthy subjects (Figure 2 left). Unsupervised machine learning was applied to identify structures in the data

$$D = \{x_i, i = 1, \ldots, 403\} \subset \mathbb{R}^d.$$ Following projection of the vector space onto a toroid grid of 20 x 80 = 4,000 neurons and training of a self-organizing map, a U-matrix visualization was displayed on top of this SOM (Figure 2 right). This provided an emergent self-organizing feature map (ESOM), in which large U-heights indicated a large gap in the data space whereas low U-heights indicated that the points are close to each other in the data space indicating structure in the data set. On the topographic map of the U-matrix, valleys, ridges and basins enhance the visibility of the structure of clusters. From this structure, a circular "mountain range" separated a region from another appearing as a green-blue colored "valley", which indicates the emergence of two main clusters in the data. Superimposing the cluster structure with the class labeling into MS patients or control indicated an almost perfect separation of the diagnostic groups by the cluster structure, with only a few patients being located in the region dominated by healthy subjects. This was reflected balanced accuracy of diagnosis assignment of 98 %. Thus, results of unsupervised machine learning supported that the set of lipid markers includes information suitable to separate MS from controls and in the subsequent steps of data analysis, the lipid markers were used to create a biomarker of MS.

**Example 4: Supervised machine-learning derived MS biomarkers**

An ABC analysis was applied on the mean decreases in classification accuracy when a particular feature was excluded from random forest building, as calculated during the feature selection step. During nested cross-validation including random forests, a mean classification error of 2 % (range (0 - 9 %) was observed. ABC analysis assigned 2, 3, 4, 5, 6, 7, 8, 9, or 10 items 2, 1, 1, 12, 26, 129, 549, 271 and 9 times to set "A", respectively. The most frequent size |{ABC set A}| = 8 lipid markers, i.e., the most profitable items for classifier building from the set of candidate features, was chosen for further analysis. The eight lipid markers that were most frequently members of the ABC set "A" comprised GluCerC16, HETE.15S, LPA20.4, biopterin, OEA, LacCerC24.1, PEA and C16Sphinganine, which belonged to set "A" 1000, 1000, 997, 867, 795, 791, 750, and 582 times, respectively. They were therefore chosen as components of the final classifier. The next frequent lipid marker, HETE.12S, was observed 553 times in set "A", the next, AEA, only 269 times, whereas 24 markers were never chosen as most profitable items. The selected features did not interfere with lipid markers for which evidence of a regulation in differential diagnoses of MS was found and therefore was not corrected based on topical expertise.

For the item sum based classifier, all possible rule values were iteratively assessed with respect to test performance measures, in particular to the sensitivity by specificity product. The best area under the sensitivity versus specificity curve was provided when a subject was assigned to the healthy subjects group at a sum of < 3 positively answered items (Table 2), else to the multiple sclerosis patients group. Most of these rules consisted of an assignment to the multiple sclerosis group if the concentration of the respective lipid mediator fell below a threshold. That is, most serum lipid mediator concentrations were reduced in multiple sclerosis. An exception from this pattern were the ceramide LacCerC24.1 and the sphingolipid C16Sphinganine which were found at comparatively higher concentrations in the multiple sclerosis patients. Finally, sub-symbolic classifiers based on random forests were created using the reduced d = 8 feature set and the complete d = 43 set of lipid markers. For all classifiers, i.e. (i) the reduced set item sum based classifier, (ii) the reduced set random forest based classifier, and (iii) the complete set random forest based classifier, the classification performance was comparably high (Table 3) with a balanced classification accuracy for belonging to the multiple sclerosis group of approximately 93 % or better.

**Table 2:** Conditions of the prediction of multiple sclerosis. A patient is likely to have multiple sclerosis if at least three of the items (rules) apply, i.e., the conditions given in the rows of the table are true:

| Lipid mediator | Threshold* |
|---|---|
| GluCerC16 | < 117.93 ng/mL |
| HETE.15S | < 0.44 ng/mL |
| LPA20.4 | < 71.35 ng/mL |
| Biopterin | < 1.51 ng/mL |
| OEA | < 1.57 ng/mL |
| LacCerC24.1 | > 10,880.38 ng/mL |
| PEA | < 1.67 ng/mL |
| C16Sphinganine | > 107.02 ng/mL |
| *: Lipid markers had been corrected for age and sex. Therefore, application of above rules to concentration measurements requires a correction as | |

$$CorrectedValue =$$

$$OriginalValue + \left(Age_{Subject} - 18\right) \cdot Slope_{Age} - \begin{cases} if\ male: MedianSexDifference \\ if\ female: 0 \end{cases}$$

The value of 18 corresponds to the minimum age in the present cohort and the further parameters of the are given in the following:

GluCerC16: $Slope_{Age}$ = 1.6263, MedianSexDifference = -10.2439,
HETE.15S: $Slope_{Age}$ = 0.0749, MedianSexDifference = 0.3065,
LPA20.4: $Slope_{Age}$ = -0.5206, MedianSexDifference = -9.8383,
biopterin: $Slope_{Age}$ = 0.0184, MedianSexDifference = 0.4211,
OEA: $Slope_{Age}$ = -0.0063, MedianSexDifference = 0.32,
LacCerC24.1: $Slope_{Age}$ = -19.0459, MedianSexDifference = -329.1148,
PEA: $Slope_{Age}$ = 0.0136, MedianSexDifference = 0.1177,
C16Sphinganine: $Slope_{Age}$ = -1.406441, MedianSexDifference = -0.1384429.

**Table 3:** Test performance measures for the correct prediction of the diagnosis "multiple sclerosis" provided by the eight-item serum lipidomics based classifier respectively biomarker, obtained during 1,000 runs using Bootstrap resampling from the test data set. The non-parametric confidence intervals spanning the 2.5th to the 97.5th percentiles of 1,000 Bootstrap resampling runs are given in parentheses. PPV: positive predictive value; NPV: negative predictive value; balanced accuracy = 0.5 · (sensitivity + specificity)

| Classifier | Sensitivity | Specificity | Balanced accuracy |
|---|---|---|---|
| **Symbolic classifier** (Fehler! Verweisquelle konnte nicht gefunden werden.) | 1 | 92.93 % (87.5 - 97.89 %) | 96.46 % (93.75 - 98.95 %) |
| **Subsymbolic classifier (random forests, reduced feature set)** | 95.65 % (92.96 - 97.01 %) | 98% (97.54 - 99 %) | 97.33 % (95.78 - 97.77 %) |
| **Subsymbolic classifier (random forests, complete feature set)** | 95.65 % (91.83 - 100 %) | 98.52 % (97.08 - 100 %) | 97.08 % (95.08 - 99.27 %) |

References:

Barthelmes et al., Induction of Experimental Autoimmune Encephalomyelitis in Mice and Evaluation of the Disease-dependent Distribution of Immune Cells in Various Tissues, J Vis Exp 111,2016

Compston and. Coles, Multiple sclerosis, Lancet 372(9648) (2008) 1502-17

Corthals, Multiple sclerosis is not a disease of the immune system, The Quarterly review of biology 86(4) (2011) 287-321

Del Boccio et al., Integration of metabolomics and proteomics in multiple sclerosis: From biomarkers discovery to personalized medicine, Proteomics. Clinical applications 10(4) (2016) 470-84

Hagman et al, Disease-associated inflammatory biomarker profiles in blood in different subtypes of multiple sclerosis: prospective clinical and MRI follow-up study, J Neuroimmunol 234(1-2) (2011) 141-7

Kohonen, Self-organized formation of topologically correct feature maps, Biol Cybernet 43 (1982) 59-69

Kurtzke, Rating neurologic impairment in multiple sclerosis: an expanded disability status scale (EDSS), Neurology 33(11) (1983) 1444-52

Mattsson et al., Elevated cerebrospinal fluid levels of prostaglandin E2 and 15-(S)-hydroxyeicosatetraenoic acid in multiple sclerosis, J Intern Med 265(4) (2009) 459-64

Rubegni et al., SPG2 mimicking multiple sclerosis in a family identified using next generation sequencing, J Neurol Sci. 375:198-202, Epub 2017 Jan 27

Zschiebsch et al., Tetrahydrobiopterin attenuates DSS-evoked colitis in mice by rebalancing redox and lipid signaling, J Crohns Colitis 29, 2016

**Claims**

1. A method for diagnosing multiple sclerosis (MS) in a subject suspected to suffer therefrom comprising the steps of:

   a) determining the amounts of at least three markers from a group of markers comprising LPA20.4, HETE15S (15-hydroxyeicosatetranoic acid), GluCerC16, LacCerC24.1, C16Sphinganine, biopterin, PEA (palmitoylethanolamine) and OEA (oleoylethanolamide) in a sample of said subject;
   b) comparing the determined amounts to a reference or to references; and
   c) diagnosing MS based on said comparison in said subject.

2. The method of claim 1, wherein at least the amounts of GluCer16, LPA20:4, and HETE15S are determined.

3. The method of claim 1, wherein at least the amounts of GluCer16, LPA20:4, and Biopterin are determined.

4. The method of claim 1, wherein at least the amounts of GluCer16, LPA20:4, Biopterin and HETE15S are determined.

5. The method of any one of claims 1 to 4, wherein said reference or said references are derived from a subject or a group of subjects known to suffer from MS.

6. The method of claim 5, wherein amounts for the at least three markers being essentially identical to the references are indicative for a subject suffering from MS.

7. The method of any one of claims 1 to 4, wherein said references are derived from a subject or a group of subjects known not to suffer from MS.

8. The method of claim 7, wherein amounts for the at least three markers which are significantly different from the references are indicative for a subject suffering from MS.

9. The method of any one of claims 1 to 8, wherein said sample is a blood, serum or plasma sample, in particular wherein said sample is a serum sample.

10. The method of any one of claims 1 to 9, wherein said subject is a mammal, preferably, a human.

11. The method of any one of claims 1 to 10, wherein said MS is MS of the relapsing-remitting type.

12. In vitro use of at least three markers from a group of markers comprising LPA20.4, HETE15S (hydroxyeicosatetranoic acid), GluCerC16, LacCerC24.1, C16Sphinganine, biopterin, PEA (palmitoylethanolamine) and OEA (oleoylethanolamide) for diagnosing MS in a subject.

13. The in vitro use of claim 12, wherein said at least three markers comprise GluCer16, LPA20:4, and HETE15S, or wherein said at least three markers comprise GluCer16, LPA20:4, and biopterin.

14. The use of claims 12 and 13, wherein at least four markers are used, and wherein said at least four markers comprise GluCer16, LPA20:4, biopterin and HETE15S.

15. The use of any one of claims 12 to 14, wherein said sample is a blood, serum or plasma sample, in particular wherein said sample is a serum sample.

Figure 1

Figure 1 cont.

Figure 1 cont.

Figure 1 cont.

Figure 2

Figure 3

Figure 3 contin.

Figure 3 contin.

Europäisches
Patentamt

European
Patent Office

Office européen
des brevets

# EUROPEAN SEARCH REPORT

Application Number

EP 17 19 4837

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JÖRN LÖTSCH ET AL: "Machine-Learned Data Structures of Lipid Marker Serum Concentrations in Multiple Sclerosis Patients Differ from Those in Healthy Subjects", INTERNATIONAL JOURNAL OF MOLECULAR SCIENCES 2012 MDPI AG CHE, vol. 18, no. 6, 7 June 2017 (2017-06-07), page 1217, XP055451143, ISSN: 1661-6596, DOI: 10.3390/ijms18061217 * p 9-10, bridging para; p 9, para 3 * | 1-15 | INV. G01N33/68 |
| X | SEXTON MICHELLE ET AL: "Cannabis use by individuals with multiple sclerosis: effects on specific immune parameters", INFLAMMOPHARMACOLOGY, KLUWER ACADEMIC PUBLISHERS, DORDRECHT, NL, vol. 22, no. 5, 19 August 2014 (2014-08-19), pages 295-303, XP035387126, ISSN: 0925-4692, DOI: 10.1007/S10787-014-0214-Z [retrieved on 2014-08-19] * p 5, para 1 * | 1-15 | |
| X | Kostas Kapinas ET AL: "Short communications", Nuclear Medicine Review, 1 January 2001 (2001-01-01), pages 107-111, XP055451205, Retrieved from the Internet: URL:https://journals.viamedica.pl/nuclear_medicine_review/article/download/15458/12275 [retrieved on 2018-02-14] p 109, col 1, past lines; * abstract * | 1,3,4, 12-14 | TECHNICAL FIELDS SEARCHED (IPC) G01N |

~~The present search report has been drawn up for all claims~~

| Place of search | Date of completion of the search | Examiner |
|---|---|---|
| Munich | 15 February 2018 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the
requirements of unity of invention and relates to several inventions or groups of inventions, namely:

1. claims: 1-15(partially)

   method for diagnosing multiple diagnosis comprising
   determining the amounts of at least 3 markers including at
   least LPA20.4
   ---

2. claims: 1-15(partially)

   method for diagnosing multiple diagnosis comprising
   determining the amounts of at least 3 markers including at
   least HETE15S
   ---

3. claims: 1-15(partially)

   method for diagnosing multiple diagnosis comprising
   determining the amounts of at least 3 markers including at
   least GluCerC16
   ---

4. claims: 1-15(partially)

   method for diagnosing multiple diagnosis comprising
   determining the amounts of at least 3 markers including at
   least GluCerC16
   ---

5. claims: 1-15(partially)

   method for diagnosing multiple diagnosis comprising
   determining the amounts of at least 3 markers including at
   least GluCerC16
   ---

6. claims: 1-15(partially)

   method for diagnosing multiple diagnosis comprising
   determining the amounts of at least 3 markers including at
   least biopterine
   ---

7. claims: 1-15(partially)

   method for diagnosing multiple diagnosis comprising
   determining the amounts of at least 3 markers including at
   least PEA
   ---

8. claims: 1-15(partially)

page 1 of 2

Europäisches
Patentamt

European
Patent Office

Office européen
des brevets

**LACK OF UNITY OF INVENTION
SHEET B**

The Search Division considers that the present European patent application does not comply with the
requirements of unity of invention and relates to several inventions or groups of inventions, namely:

method for diagnosing multiple diagnosis comprising
determining the amounts of at least 3 markers including at
least OEA
- - -

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4540884 A
- US 5397894 A

### Non-patent literature cited in the description

- **COMPSTON ; COLES.** Multiple sclerosis. *Lancet,* 2008, vol. 372 (9648), 1502-17
- **RUBEGNI et al.** SPG2 mimicking multiple sclerosis in a family identified using next generation sequencing. *J Neurol Sci.,* vol. 375, 198-202
- **DEL BOCCIO et al.** Integration of metabolomics and proteomics in multiple sclerosis: From biomarkers discovery to personalized medicine, Proteomics. *Clinical applications,* 2016, vol. 10 (4), 470-84
- **HAGMAN et al.** Disease-associated inflammatory biomarker profiles in blood in different subtypes of multiple sclerosis: prospective clinical and MRI follow-up study. *J Neuroimmunol,* 2011, vol. 234 (1-2), 141-7
- **CORTHALS.** Multiple sclerosis is not a disease of the immune system. *The Quarterly review of biology,* 2011, vol. 86 (4), 287-321
- **MATTSSON et al.** Elevated cerebrospinal fluid levels of prostaglandin E2 and 15-(S)-hydroxyeicosatetraenoic acid in multiple sclerosis. *J Intern Med,* 2009, vol. 265 (4), 459-64
- **BARTHELMES et al.** Induction of Experimental Autoimmune Encephalomyelitis in Mice and Evaluation of the Disease-dependent Distribution of Immune Cells in Various Tissues. *J Vis Exp,* 2016, vol. 111
- *Int. J. Mol. Sci.,* 2017, vol. 18, 1217
- **DOWDY ; WEARDEN.** Statistics for Research. John Wiley & Sons, 1983
- **NISSEN.** *Journal of Chromatography A,* 1995, vol. 703, 37-57
- **ZWEIG MH. et al.** *Clin. Chem.,* 1993, vol. 39, 561-577
- **KURTZKE.** Rating neurologic impairment in multiple sclerosis: an expanded disability status scale (EDSS). *Neurology,* 1983, vol. 33 (11), 1444-52
- **ZSCHIEBSCH et al.** Tetrahydrobiopterin attenuates DSS-evoked colitis in mice by rebalancing redox and lipid signaling. *J Crohns Colitis,* 2016, vol. 29
- **KOHONEN.** Self-organized formation of topologically correct feature maps. *Biol Cybernet,* 1982, vol. 43, 59-69